# EUROPEAN PATENT APPLICATION

(11) **EP 3 806 457 A1**
(43) Date of publication of application: **14.04.2021**
(21) Application number: 19811717.8
(22) Date of filing: 12.03.2019
(51) Int. Cl.: H04N 13/128, A61B 1/00, A61B 90/20, G02B 27/22, G03B 35/08, G03B 35/18, H04N 5/232, H04N 13/239, H04N 13/356, H04N 13/361

(54) **MEDICAL IMAGE PROCESSING APPARATUS**

(30) Priority: 30.05.2018 JP 2018102993
(71) Applicant: Sony Olympus Medical Solutions Inc., Hachioji-shi, Tokyo 192-0904 (JP)
(72) Inventor: KOBAYASHI, Motoaki, Hachioji-shi, Tokyo 192-0904 (JP)
(74) Representative: D Young & Co LLP
(86) International application number: PCT/JP2019/009922
(87) International publication number: WO 2019/230115

(57) **Abstract**

Provided is a medical image processing apparatus capable of obtaining a medical image with a more natural three-dimensional effect.

Provided is a medical image processing apparatus including: an image processor configured to set a cut-out range for each of a medical image for a right eye and a medical image for a left eye, captured respectively by a first imaging device and a second imaging device with a fixed inward angle that is an angle formed by imaging directions, and perform an electronic zoom process of enlarging an image in the cut-out range; and a display controller configured to control display on a display screen of the medical image for the right eye and the medical image for the left eye that have been subjected to the electronic zoom process by the image processor, wherein the image processor is configured to set the cut-out range for each of the medical image for the right eye and the medical image for the left eye based on a reference distance corresponding to the inward angle and a subject distance in the first imaging device and the second imaging device.

## Description

### Field

The present disclosure relates to a medical image processing apparatus.

### Background

In recent years, there is a case, in medical fields, of using a medical observation apparatus capable of enlarged observation of an observation target such as an affected site in order to support microsurgery such as neurosurgery. Examples of the medical observation apparatus include a medical observation apparatus equipped with an optical microscope and a medical observation apparatus equipped with an imaging device that functions as an electronic imaging microscope. Hereinafter, the medical observation apparatus equipped with the above-described optical microscope will be referred to as an "optical medical observation apparatus". In addition, hereinafter, a medical observation apparatus equipped with the above-described imaging device will be referred to as an "electronic imaging medical observation apparatus" or simply as a "medical observation apparatus".

With increased image quality in imaging devices and display devices that display captured images, an electronic imaging medical observation apparatus can obtain image quality equal to or higher than that of an optical medical observation apparatus. In addition, a user of an electronic imaging medical observation apparatus (for example, a medical worker such as a practitioner or an assistant of the practitioner) need not observe through an eyepiece of an optical microscope as in the case of using an optical medical observation apparatus, and thus it is possible to more freely move the position of the imaging device. Therefore, the use of the electronic imaging medical observation apparatus has an advantage that surgical operations can be supported more flexibly by moving the position of the imaging device, promoting the use of the electronic imaging medical observation apparatus in medical fields.

Under such circumstances, technologies concerning an electronic imaging medical observation apparatus have been developed. Examples of the above technologies include the technology described in Patent Literature 1 below.

### Citation List

### Patent Literature

Patent Literature 1: JP 2006-218105 A

### Summary

### Technical Problem

Some medical observation apparatuses include a plurality of imaging devices and have a function of capturing an image for the right eye and an image for the left eye, that is, can capture a 3D image. Hereinafter, an image captured by a medical observation apparatus is referred to as a "medical image".

In addition, some display devices capable of displaying a medical image have a configuration corresponding to any types of display capable of 3D display, such as an eyeglass type that enables 3D display in cooperation with an eyeglass-type device, or a naked eye type that enables 3D display without cooperation with the eyeglass-type device. With the medical image for the right eye and the medical image for the left eye displayed on individual display screens of display devices, a viewer of the display screen can obtain a three-dimensional effect on the displayed medical images.

Here, in order to reproduce a natural three-dimensional effect with less fatigue, it is desirable to achieve a match between an inward angle (described below) in two imaging devices, that is, the device for capturing a medical image for the right eye and the device for capturing a medical image for the left eye and a convergence angle (described below) formed by the right eye and the left eye of the viewer of the display screen.

However, assuming a situation where the medical observation apparatus and the display device are used, the convergence angle (described below) varies from display device to display device due to various factors such as a location where the display device is arranged and a size of the display screen. Therefore, even if the inward angle (described below) in the medical observation apparatus is fixed, it is difficult to achieve a match between the inward angle (described below) and the convergence angle (described below). The greater the difference between the inward angle (described below) and the convergence angle (described below), the more the viewer (hereinafter, referred to as an "observer") of the medical image displayed on the display screen feels that the image is a flat image with no three-dimensional effect, or that the image is a distorted image with an excessive three-dimensional effect.

The present disclosure proposes a novel and improved medical image processing apparatus capable of obtaining a medical image with a more natural three-dimensional effect.

### Solution to Problem

According to the present disclosure, there is provided a medical image processing apparatus including: an image processor configured to set a cut-out range for each of a medical image for a right eye and a medical image for a left eye, captured respectively by a first imaging device and a second imaging device with a fixed inward angle that is an angle formed by imaging directions, and perform an electronic zoom process of enlarging an image in the cut-out range; and a display controller configured to control display on a display screen of the medical image for the right eye and the medical image for the left eye that have been subjected to the electronic zoom process by the image processor, wherein the image processor is configured to set the cut-out range for each of the medical image for the right eye and the medical image for the left eye based on a reference distance corresponding to the inward angle and a subject distance in the first imaging device and the second imaging device.

### Advantageous Effects of Invention

According to the present disclosure, it is possible to obtain a medical image with a more natural three-dimensional effect.

Note that the effect is not necessarily limitative and thus, in addition to the effect or instead of the effect, any effect in the present specification or other effects to be grasped from the present specification may be produced.

### Brief Description of Drawings

FIG. 1 is an explanatory view for describing an inward angle and a convergence angle.
FIG. 2 is a view illustrating an example of a medical observation apparatus according to the present embodiment.
FIG. 3 is an explanatory view illustrating an example of a hardware configuration of the medical observation apparatus according to the present embodiment.
FIG. 4 is an explanatory view illustrating the example of the hardware configuration of the medical observation apparatus according to the present embodiment.
FIG. 5 is an explanatory view illustrating the example of the hardware configuration of the medical observation apparatus according to the present embodiment.
FIG. 6 is a functional block diagram illustrating an example of a configuration of a medical observation apparatus according to the present embodiment.
FIG. 7 is an explanatory view for describing an example of an electronic zoom process in the medical observation apparatus according to the present embodiment.
FIG. 8 is an explanatory view illustrating a first example of setting of a cut-out range in the medical observation apparatus according to the present embodiment.
FIG. 9 is an explanatory view illustrating a second example of setting of the cut-out range in the medical observation apparatus according to the present embodiment.
FIG. 10 is an explanatory view illustrating a third example of setting of the cut-out range in the medical observation apparatus according to the present embodiment.
FIG. 11 is an explanatory view for describing an example of the electronic zoom process in the medical observation apparatus according to the present embodiment.
FIG. 12 is an explanatory view for describing an example of the electronic zoom process in the medical observation apparatus according to the present embodiment.
FIG. 13 is an explanatory view for describing an example of a display control process in the medical observation apparatus according to the present embodiment.
FIG. 14 is an explanatory view for describing a first example of display performed by the display control process in the medical observation apparatus according to the present embodiment.
FIG. 15 is an explanatory view for describing a second example of display performed by the display control process in the medical observation apparatus according to the present embodiment.
FIG. 16 is an explanatory view for describing a third example of display performed by the display control process in the medical observation apparatus according to the present embodiment.
FIG. 17 is an explanatory view for describing the third example of display performed by the display control process in the medical observation apparatus according to the present embodiment.

### Description of Embodiments

A preferred embodiment of the present disclosure will be described in detail below with reference to the accompanying drawings. Note that, in the present specification and the drawings, constituent elements having substantially the same functional configurations are denoted with the same reference signs, and the duplicate descriptions thereof will be omitted.

Hereinafter, description will be given in the following order.
1. Medical Observation Apparatus According to Present Embodiment
   [1] Outline of Medical Observation Apparatus According to Present Embodiment
   [2] Configuration Example of Medical Observation Apparatus According to Present Embodiment
   [3] Example of processing in Medical Observation Apparatus according to Present Embodiment
   [4] Example of Effect Achieved by Using Medical Observation Apparatus According to Present Embodiment
2. Program According to Present Embodiment

### (Medical Observation Apparatus According to Present Embodiment)

### [1] Outline of Medical Observation Apparatus According to Present Embodiment

A medical observation apparatus according to the present embodiment (hereinafter, sometime simply referred to as the "medical observation apparatus") is an apparatus including at least two imaging devices capable of capturing a 3D image and a display device capable of 3D display. Note that the medical observation apparatus is also a medical image processing apparatus. Hereinafter, a case where the medical observation apparatus includes an imaging device that captures a medical image for the right eye and an imaging device that captures a medical image for the left eye will be described as an example.

As described above, it is desirable to achieve a match between an inward angle in the two imaging devices and a convergence angle formed by the right eye and the left eye of an observer in order to reproduce a natural three-dimensional effect with less fatigue.

The inward angle according to the present embodiment is "an angle formed by an imaging direction in an imaging device that captures a medical image for the right eye and an imaging direction in an imaging device that captures a medical image for the left eye". In other words, the inward angle is the angle formed by center lines of imaging planes of the respective imaging devices with respect to a tolerance point. Hereinafter, an imaging device that captures the medical image for the right eye is referred to as a "first imaging device", and an imaging device that captures the medical image for the left eye is referred to as a "second imaging device".

In the medical observation apparatus, the imaging direction of the first imaging device and the imaging direction of the second imaging device are fixed, and as a result, the inward angle is fixed as illustrated in a configuration example described below.

The convergence angle according to the present embodiment is an "angle between the right eye and the left eye of the observer". The convergence angle corresponds to "an angle formed between the line of sight of the right eye and the line of sight of the left eye when a certain position on the display screen is in focus", for example. The convergence angle corresponding to the display device can be estimated from a representative value (for example, an average value) of human's interocular distance and a set value of the distance (viewing distance) between the observer and the display screen. Note that the convergence angle corresponding to the display device may be set to a value corresponding to a specific person on the basis of an interocular distance value set for a specific person and a detection result of a distance (viewing distance) between the specific person and the display screen detected by a distance sensor or the like.

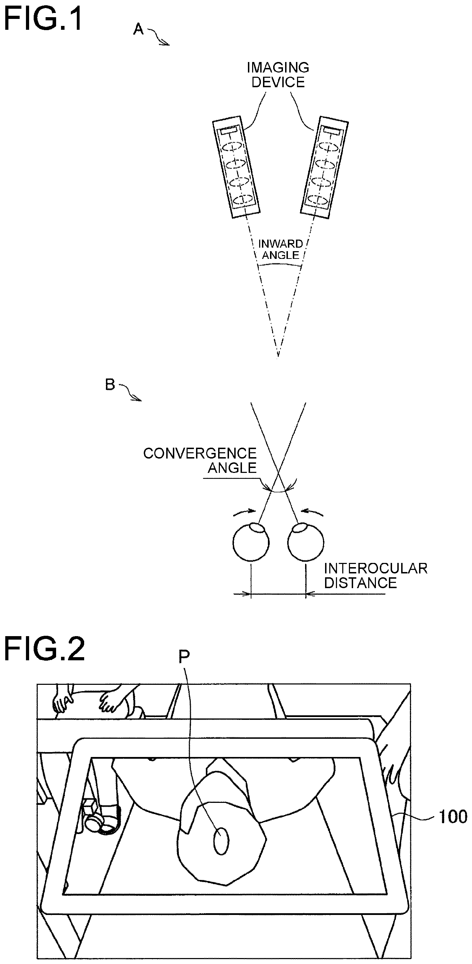

FIG. 1 is an explanatory view for describing the inward angle and the convergence angle. A of FIG. 1 conceptually illustrates the inward angle, and B of FIG. 1 conceptually illustrates the convergence angle. The interocular distance illustrated in B of FIG. 1 is generally about 60 to 70 [mm].

As described above, the greater the difference between the inward angle and the convergence angle, the more the viewer of the medical image displayed on the display screen feels that the image is a flat image with no three-dimensional effect or that the image is a distorted image with an excessive three-dimensional effect. More specifically, the greater the difference between the inward angle and the convergence angle, the more the perception in the depth direction changes. In addition, the greater the viewing distance, the greater the sense of depth perceived.

Therefore, it is desirable to achieve a match between the inward angle and the convergence angle in order to give a more natural three-dimensional effect to the observer of the medical image displayed on the display screen of the display device. When a match between the inward angle and the convergence angle is achieved, it is possible to achieve a more precise view with respect to the horizontal direction and the depth direction, enabling acquisition of a more natural three-dimensional effect.

However, it is difficult to achieve a match between the inward angle and the convergence angle even if the inward angle in the medical observation apparatus is fixed as described above.

In addition, for example, when an imaging device having "a structure for adjusting an inward angle by changing an angle of a mirror or a prism" is provided as in an apparatus to which the technique described in Patent Literature 1 is applied, the apparatus becomes large, heavy, and expensive.

Therefore, the medical observation apparatus has a configuration for fixing the inward angle, and controls a cut-out range in an electronic zoom process to acquire a medical image that gives a more natural three-dimensional effect.

### [2] Configuration Example of Medical Observation Apparatus According to Present Embodiment

Next, a configuration example of the medical observation apparatus according to the present embodiment will be described.

FIG. 2 is an explanatory view illustrating an example of a medical observation apparatus 100 according to the present embodiment, and illustrates an example of a surgical operation field in which the medical observation apparatus 100 is used. FIG. 2 illustrates an example of a view seen from an observer who is operating a surgery on an affected site P using the medical observation apparatus 100.

### [2-1] Hardware Configuration Example of Medical Observation Apparatus According to Present Embodiment

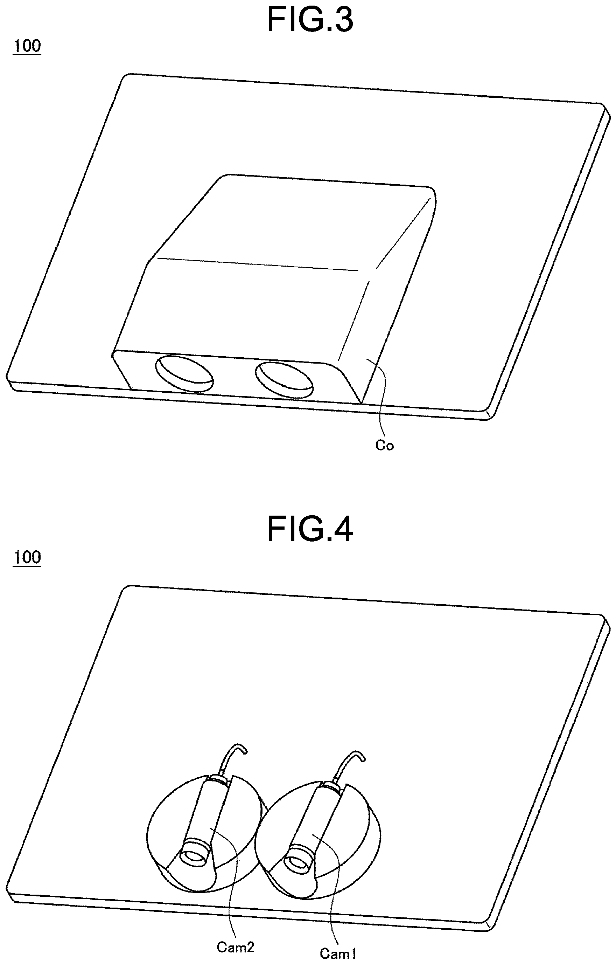

FIGS. 3 to 5 are explanatory views illustrating an example of a hardware configuration of the medical observation apparatus 100 according to the present embodiment.

FIG. 3 is a perspective view of the medical observation apparatus 100 viewed from the back side (the side opposite to the display screen). A cover member Co is provided on the back side of the medical observation apparatus 100. The cover member Co is detachable.

FIG. 4 is a perspective view illustrating the medical observation apparatus 100 in a state where the cover member Co illustrated in FIG. 3 has been removed. As illustrated in FIGS. 3 and 4, a first imaging device Cam1 and a second imaging device Cam2 are provided inside the cover member Co. The first imaging device Cam1 and the second imaging device Cam2 are arranged such that imaging directions are fixed, and an inward angle is fixed in the medical observation apparatus 100.

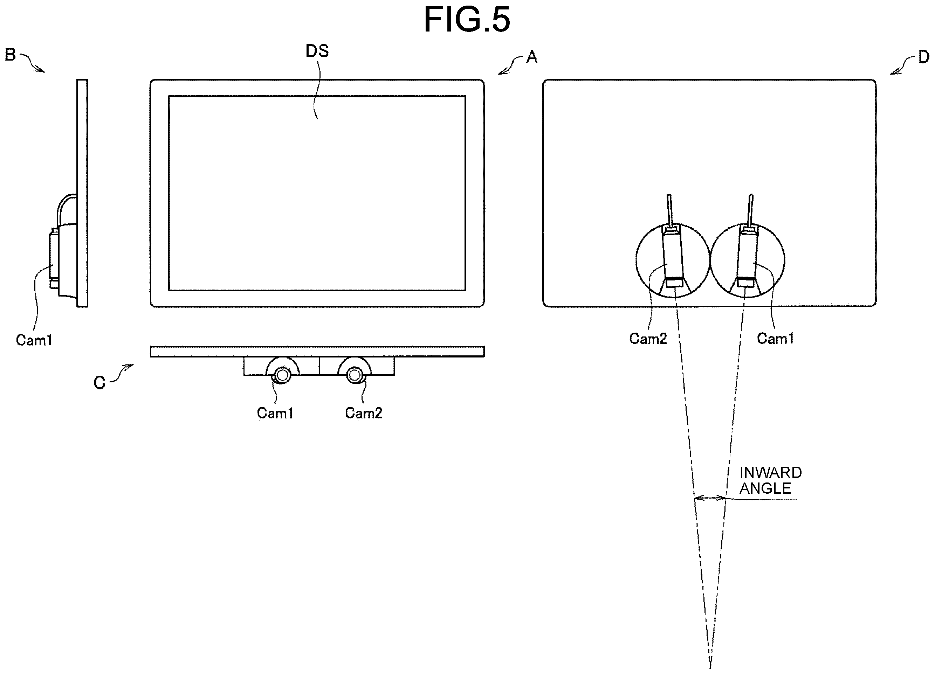

FIG. 5 is an explanatory view illustrating an example of an external appearance of the medical observation apparatus 100 in the state where the cover member Co illustrated in FIG. 3 has been removed. A of FIG. 5 is a front view of the medical observation apparatus 100 viewed from the front side (a side of a display screen DS). B of FIG. 5 and C of FIG. 5 are side views of the medical observation apparatus 100, and D of FIG. 5 is a rear view of the medical observation apparatus 100. Note that it goes without saying that the external appearance of the medical observation apparatus 100 is not limited to the example illustrated in FIG. 5. Hereinafter, the medical observation apparatus 100 in the state where the cover member Co has been removed as illustrated in FIGS. 4 and 5 is taken as an example for convenience of description.

Hereinafter, the example of the hardware configuration of the medical observation apparatus 100 will be described. The medical observation apparatus 100 is driven by electric power supplied from an internal power supply such as a battery included in the medical observation apparatus 100 or by electric power supplied from a connected external power supply, for example.

As illustrated in FIGS. 4 and 5, the medical observation apparatus 100 includes two imaging devices of, for example, the first imaging device Cam1 and the second imaging device Cam2. Note that the medical observation apparatus according to the present embodiment may include three or more imaging devices as described above.

As the first imaging device Cam1 and the second imaging device Cam2, it is possible to apply configurations of imaging devices used in various known electronic imaging microscope units.

As an example, each of the first imaging device Cam1 and the second imaging device Cam2 includes an optical system and an image sensor that includes an imaging element that captures an image of an observation target using light that has passed through the optical system, for example. For example, the optical system includes, as optical elements, an objective lens, at least one lens of a zoom lens and a focusing lens, and a mirror. Examples of the image sensor include an image sensor using a plurality of imaging elements such as a complementary metal oxide semiconductor (CMOS) and a charge coupled device (CCD).

Each of the first imaging device Cam1 and the second imaging device Cam2 is equipped with one or more functions generally provided in an electronic imaging microscope unit such as a zoom function including at least an electronic zoom function (electronic zoom function or an optical zoom function and an electronic zoom function) and an auto focus (AF) function.

Each of the first imaging device Cam1 and the second imaging device Cam2 may be configured to be capable of imaging at a so-called high resolution such as 4K and 8K. With the configuration in which each of the first imaging device Cam1 and the second imaging device Cam2 is capable of high-resolution imaging, it is possible to display an image on the display device having a large display screen of 50 inches or more, for example, while ensuring a predetermined resolution (full HD image quality, for example). Accordingly, the visibility of the observer such as an operator is improved. In addition, it is possible to ensure a predetermined resolution even if the medical image is enlarged by the electronic zoom function and displayed on the display screen of the display device since each of the first imaging device Cam1 and the second imaging device Cam2 is capable of imaging at a high resolution. Furthermore, when the predetermined resolution is ensured using the electronic zoom function, it is possible to suppress the performance of the optical zoom function in each of the first imaging device Cam1 and the second imaging device Cam2. Accordingly, the optical system of each of the first imaging device Cam1 and the second imaging device Cam2 can be made simpler, and each of the first imaging device Cam1 and the second imaging device Cam2 can be further downsized.

In addition, the medical observation apparatus 100 includes the display device capable of displaying an arbitrary image on the display screen DS as illustrated in FIG. 5. Examples of the display device include a liquid crystal display and an organic electro-luminescence (EL) display.

Although not illustrated in FIGS. 3 to 5, the medical observation apparatus 100 includes, for example, one processor or two or more processors (not illustrated) each of which is constituted by an arithmetic circuit such as a micro processing unit (MPU), a read only memory (ROM; not illustrated), a random access memory (RAM; not illustrated), a recording medium (not illustrated), and a communication device (not illustrated).

The processor (not illustrated) functions as a control unit described below. The ROM (not illustrated) stores programs used by the processor (not illustrated) and control data such as calculation parameters. The RAM (not illustrated) temporarily stores a program executed by the processor (not illustrated), or the like.

The recording medium (not illustrated) functions as a storage unit (not illustrated) in the medical observation apparatus 100. In the recording medium (not illustrated), for example, various types of data such as "data relating to various processes in the medical observation apparatus 100 such as data indicating a reference distance (described below) corresponding to the inward angle" and various applications are stored. Here, examples of the recording medium (not illustrated) include a magnetic recording medium such as a hard disk, and nonvolatile memory such as flash memory. Furthermore, the recording medium (not illustrated) may be removable from the medical observation apparatus 100.

The communication device (not illustrated) is a communication unit included in the medical observation apparatus 100 and has a role of performing wireless or wired communication with an external device. Here, examples of the communication device (not illustrated) include an IEEE 802.15.1 port and a transmission/reception circuit (wireless communication), an IEEE 802.11 port and a transmission/reception circuit (wireless communication), a communication antenna and a radio frequency (RF) circuit (wireless communication), or a local area network (LAN) terminal and a transmission/reception circuit (wired communication).

The medical observation apparatus 100 has, for example, the hardware configuration described above. Note that it goes without saying that the hardware configuration of the medical observation apparatus 100 is not limited to the above example. For example, the medical observation apparatus 100 may be realized as a single apparatus including all the configurations described above, or some or all of the configurations may be realized as separate apparatuses. As an example, the first imaging device Cam1 and the second imaging device Cam2 (an imaging unit described below) may be realized as independent devices using imaging devices or the like independent of the medical observation apparatus 100. In addition, the display device (a display unit described below) may be realized as a single device using a display device or the like independent of the medical observation apparatus 100. In addition, the medical observation apparatus 100 may be a medical image processing apparatus including at least a processor (a control unit described below).

### [2-2] Functional Configuration of Medical Observation Apparatus 100

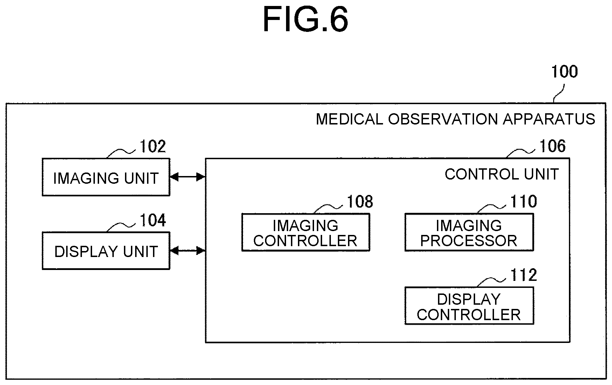

Next, the medical observation apparatus 100 illustrated in FIGS. 3 to 5 will be described using functional blocks. FIG. 6 is a functional block diagram illustrating an example of the configuration of the medical observation apparatus 100 according to the present embodiment.

The medical observation apparatus 100 includes, for example, an imaging unit 102, a display unit 104, and a control unit 106.

The imaging unit 102 captures an image of an observation target. The imaging unit 102 is constituted by two imaging devices, for example, the first imaging device Cam1 and the second imaging device Cam2. The imaging by the imaging unit 102 is controlled by, for example, the control unit 106.

The display unit 104 is a display means provided in the medical observation apparatus 100, and displays various images such as a medical image on a display screen. The display unit 104 is configured by, for example, the display device described above. The display on the display unit 104 is controlled by the control unit 106, for example.

The control unit 106 includes the above-described processor (not illustrated), for example, and has a role of controlling the entire medical observation apparatus 100. In addition, the control unit 106 plays a leading role in processes in the medical observation apparatus described below. Note that the processing in the control unit 106 may be distributed among a plurality of processing circuits (for example, a plurality of processors) to be executed.

More specifically, the control unit 106 includes, for example, an imaging controller 108, an image processor 110, and a display controller 112.

The imaging controller 108 controls the imaging device forming the imaging unit 102. Examples of the control on the imaging device include control on one or more general functions of an electronic imaging type microscope unit such as control on an AF function at least including a zoom function (the electronic zoom function or the optical zoom function and the electronic zoom function).

The image processor 110 performs an electronic zoom process on each of a medical image for the right eye and a medical image for the left eye. The electronic zoom process according to the present embodiment is a process of setting a cut-out range for a medical image and enlarging an image in the cut-out range. An example of the electronic zoom process according to the present embodiment will be described below.

The display controller 112 performs a display control process of displaying a medical image on the display screen. The display controller 112 controls the display of the display device by transmitting a display control signal and an image signal to the display device forming the display unit 104, for example.

Examples of display control of a medical image on the display screen, which is realized by the display control process in the display controller 112, include the following examples. An example of the display control process according to the present embodiment will be described below.
(a) Control to display each of a medical image for the right eye and a medical image for the left eye on the display screen (3D display control)
(b) Control to display one of a medical image for the right eye and a medical image for the left eye (2D display control)
(c) Control to display an image that has been subjected the electronic zoom process on the display screen
(d) Combination of the above (a) or the above (b) and the above (c)

Note that the functional configuration of the control unit 106 is not limited to the example illustrated in FIG. 6.

For example, when the display control process is performed in an apparatus outside the medical observation apparatus 100, such as a medical controller, the control unit 106 does not necessarily include the display controller 112.

In addition, the control unit 106 may be arbitrarily configured according to a way of dividing the functions of the medical observation apparatus 100 such as a configuration according to a way of dividing the processes performed in the medical observation apparatus 100.

The medical observation apparatus 100 has, for example, a functional configuration illustrated in FIG. 6. Note that the functional configuration of the medical observation apparatus according to the present embodiment is not limited to the configuration illustrated in FIG. 6.

For example, the medical observation apparatus according to the present embodiment can implement a part or all of the imaging controller 108, the image processor 110, and the display controller 112 illustrated in FIG. 6 separately from the control unit 106 (for example, using another processing circuit).

In addition, when the display control process is performed in the apparatus outside the medical observation apparatus 100, the medical observation apparatus according to the present embodiment does not necessarily include the display controller 112.

### [3] Example of processing in Medical Observation Apparatus according to Present Embodiment

Next, the electronic zoom process and the display control process will be described as examples of the processing in the medical observation apparatus according to the present embodiment. Hereinafter, a case where the medical observation apparatus 100 performs the electronic zoom process and the display control process will be described as an example. Note that the electronic zoom process in the medical observation apparatus 100 is performed, for example, in the image processor 110 provided in the control unit 106 as described above. In addition, the display control process in the medical observation apparatus 100 is performed, for example, in the display controller 112 provided in the control unit 106 as described above.

### [3-1] Electronic Zoom Process According to Present Embodiment

First, the electronic zoom process according to the present embodiment will be described. As described above, the electronic zoom process is a process of setting a cut-out range for a medical image and enlarging an image in the cut-out range. The medical observation apparatus 100 enlarges the image in the cut-out range at a set magnification. The set magnification may be a preset fixed value or may be a variable value that can be changed by an operation of a user of the medical observation apparatus 100 or the like. In addition, the magnification related to the electronic zoom process may be set such that a display parallax amount in the enlarged image of the cut-out range approaches a display parallax amount corresponding to a distance from an observer viewing the display screen to a subject as will be described below.

The medical observation apparatus 100 sets the cut-out range for each of the medical image for the right eye and the medical image for the left eye based on the reference distance corresponding to the inward angle and a subject distance in the first imaging device Cam1 and the second imaging device Cam2.

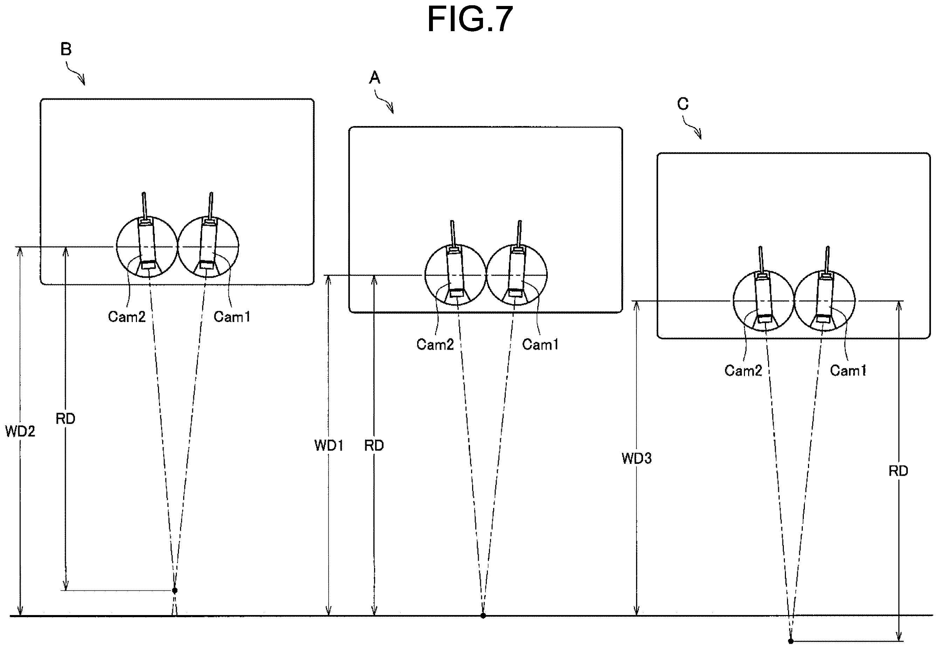

FIG. 7 is an explanatory view for describing an example of the electronic zoom process in the medical observation apparatus 100 according to the present embodiment. "RD" illustrated in FIG. 7 illustrates an example of the reference distance corresponding to the inward angle (hereinafter, the same applies to other drawings). Each of "WD1", "WD2", and "WD3" illustrated in FIG. 7 illustrates an example of the subject distance. Hereinafter, the reference distance may be referred to as "reference distance RD" and the subject distance may be referred to as "subject distance WD".

The reference distance RD is a "distance between a reference position of each of the first imaging device Cam1 and the second imaging device Cam2 and a position of an intersection of the imaging direction in the first imaging device Cam1 and the imaging direction in the second imaging device Cam2". Since the inward angle is fixed in the medical observation apparatus 100, the reference distance RD may be, for example, a fixed value associated with the inward angle. Note that the reference distance RD may be a variable value that can be changed by the operation of the user of the medical observation apparatus 100. Hereinafter, the intersection is referred to as a "focus point".

The subject distance WD is a distance called a working distance. Examples of the subject distance WD may include a "distance between the reference position of each of the first imaging device Cam1 and the second imaging device Cam2 and a subject, such as an affected site, when the focus is achieved". The position of the subject when the focus is achieved is called, for example, an in-focus position. The subject distance WD is identified by any method, for example, using a distance measurement result obtained by a distance sensor (not illustrated) (distance measured by the distance sensor) or calculating a hyperfocal distance in the first imaging device Cam1 or the second imaging device Cam2. The distance sensor (not illustrated) may be a distance sensor provided in the medical observation apparatus 100, or may be an external distance sensor connected to the medical observation apparatus 100.

A of FIG. 7 illustrates a case where the reference distance RD and the subject distance WD match. B of FIG. 7 is an example of a case where the reference distance RD and the subject distance WD do not match, and illustrates a case where the subject distance WD is longer than the reference distance RD. C of FIG. 7 is another example of the case where the reference distance RD and the subject distance WD do not match, and illustrates a case where the subject distance WD is shorter than the reference distance RD.

Since the inward angle is fixed in the medical observation apparatus 100, it is difficult to obtain an image (an example of a medical image) that has been subjected to the electronic zoom process with a more natural three-dimensional effect in a case where the cut-out range is set to be the same in each of the state A of FIG. 7, the state B of FIG. 7, and the state C of FIG. 7.

Therefore, the medical observation apparatus 100 controls the setting of the cut-out range for each of the medical image for right eye and the medical image for left eye based on the reference distance RD and the subject distance WD.

Specifically, the medical observation apparatus 100 sets the cut-out range based on a difference between the reference distance RD and the subject distance WD. Hereinafter, the difference between the reference distance RD and the subject distance WD is sometimes simply referred to as the "difference". In addition, a case where the difference is a value obtained by subtracting the subject distance WD from the reference distance RD will be described hereinafter as an example. Note that the difference may be a value obtained by subtracting the reference distance RD from the subject distance WD.

The medical observation apparatus 100 identifies an adjustment amount corresponding to a difference by referring to, for example, a "table (or database) in which the difference and the adjustment amount of the cut-out range are associated". In addition, the medical observation apparatus 100 may identify an adjustment amount corresponding to a difference by an operation of an arbitrary algorithm that can identify the adjustment amount of the cut-out range based on the difference. An example of the adjustment amount according to the present embodiment is the number of pixels. Note that the adjustment amount according to the present embodiment may be any value that can shift the cut-out range from the reference position. The above-described table is stored in a recording medium that functions as a storage unit (not illustrated) or an external recording medium.

Examples of the reference position of the cut-out range according to the present embodiment include a position at which an image that gives a more natural three-dimensional effect can be obtained when the reference distance RD and the subject distance WD match, that is, a position where a center position of the cut-out range becomes a center position of a medical image. Note that it goes without saying that the reference position of the cut-out range according to the present embodiment is not limited to the above example. Hereinafter, a case where the reference position of the cut-out range is "the position where the center position of the cut-out range becomes the center position of the medical image" will be described as an example.

Then, the medical observation apparatus 100 sets a cut-out range such that a center position of the cut-out range is moved in the horizontal direction by pixels corresponding to a difference from the center position of each of a medical image for the right eye and a medical image for the left eye.

Hereinafter, examples of setting a cut-out range in each of the state A of FIG. 7, the state B of FIG. 7, and the state C of FIG. 7 will be described.

### (1) First example of setting cut-out range: Setting example when reference distance RD and subject distance WD match

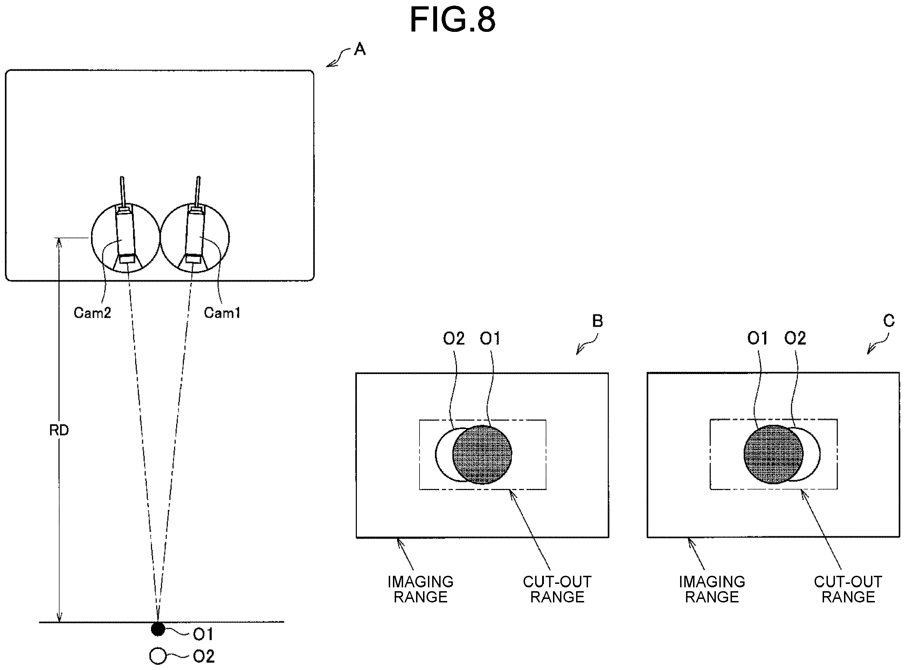

FIG. 8 is an explanatory view illustrating a first example of setting of a cut-out range in the medical observation apparatus 100 according to the present embodiment. A of FIG. 8 illustrates a case where subjects O1 and O2 are captured in the state illustrated in A of FIG. 7. B of FIG. 8 illustrates a medical image for the left eye obtained in the case of A of FIG. 8, and C of FIG. 8 illustrates a medical image for the right eye obtained in the case of A of FIG. 8.

In the state illustrated in A of FIG. 8, the reference distance RD and the subject distance WD match, and the difference is zero. In this case, the subject O1 at the focus point is at the center of the image for both the medical image for the left eye and the medical image for the right eye.

Accordingly, in the case of the state illustrated in A of FIG. 8, the medical observation apparatus 100 sets the cut-out range such that the center position of the cut-out range becomes the center position of each of the medical image for the right eye and the medical image for the left eye as illustrated in B of FIG. 8 and C of FIG. 8.

### (2) Second example of setting cut-out range: Example of setting when reference distance RD and subject distance WD do not match

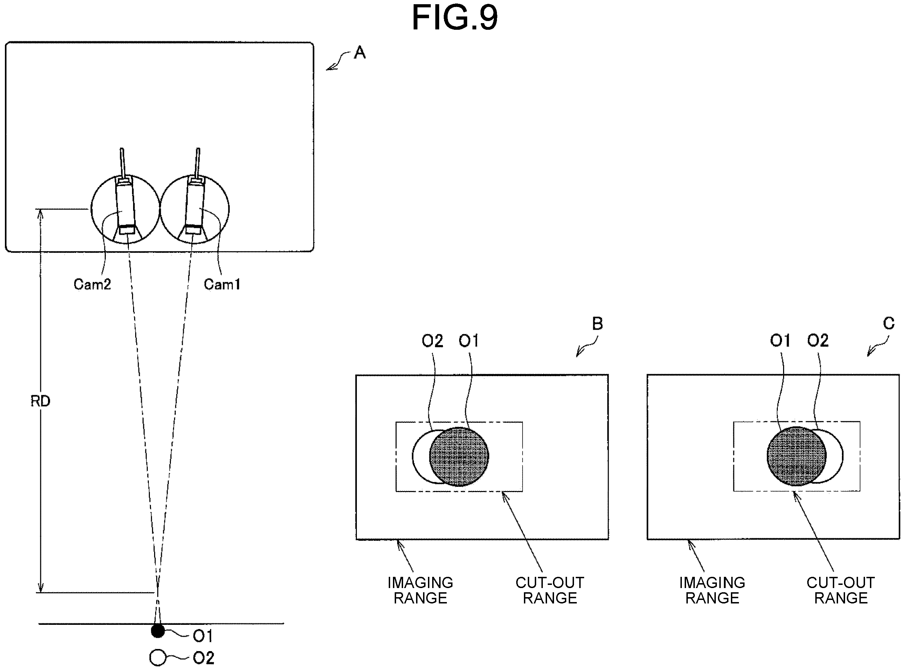

FIG. 9 is an explanatory view illustrating a second example of setting of a cut-out range in the medical observation apparatus 100 according to the present embodiment. A of FIG. 9 illustrates a case where the subjects O1 and O2 are captured in the state illustrated in B of FIG. 7. B of FIG. 9 illustrates a medical image for the left eye obtained in the case of A of FIG. 9, and C of FIG. 9 illustrates a medical image for the right eye obtained in the case of A of FIG. 9.

In the state illustrated in A of FIG. 9, the reference distance RD and the subject distance WD do not match, and the difference is a value other than zero (for example, a negative value). In this case, the subject O1 is not at the center of the image for both the medical image for the left eye and the medical image for the right eye.

Accordingly, in the case of the state illustrated in A of FIG. 9, the medical observation apparatus 100 sets the cut-out range to move in the horizontal direction by pixels corresponding to the difference from the center position of each of the medical image for the right eye and the medical image for the left eye as illustrated in B of FIG. 9 and C of FIG. 9.

Since the cut-out range corresponding to the difference is set as described above, it is possible to obtain an image in a state where a match is achieved between the reference distance RD and the subject distance WD even if the inward angle is fixed. Accordingly, the "image obtained by cutting out the cut-out range and enlarging the cut-out range at the set magnification", that is, the image that has been subjected to the electronic zoom process becomes an image with a more natural three-dimensional effect.

### (3) Third example of setting of cut-out range: Another example of setting when reference distance RD and subject distance WD do not match

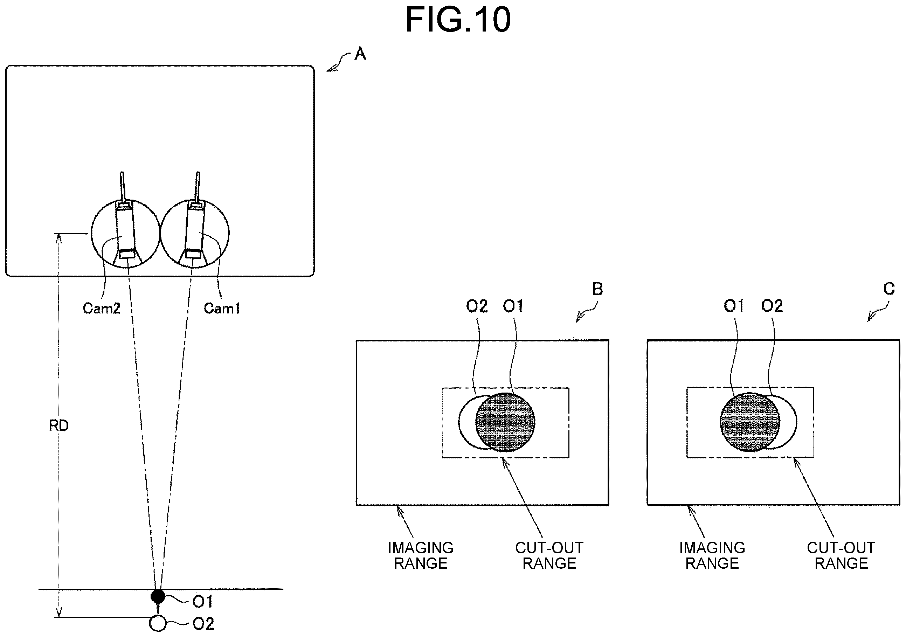

FIG. 10 is an explanatory view illustrating a third example of setting of a cut-out range in the medical observation apparatus 100 according to the present embodiment. A of FIG. 10 illustrates a case where subjects O1 and O2 are captured in the state illustrated in C of FIG. 7. B of FIG. 10 illustrates a medical image for the left eye obtained in the case of A of FIG. 10, and C of FIG. 10 illustrates a medical image for the right eye obtained in the case of A of FIG. 10.

In the state illustrated in A of FIG. 10, the reference distance RD and the subject distance WD do not match, and the difference is a value other than zero (for example, a positive value). In this case, the subject O1 is not at the center of the image for both the medical image for the left eye and the medical image for the right eye.

Accordingly, in the case of the state illustrated in A of FIG. 10, the medical observation apparatus 100 sets the cut-out range to move in the horizontal direction by pixels corresponding to the difference from the center position of each of the medical image for the right eye and the medical image for the left eye as illustrated in B of FIG. 10 and C of FIG. 10.

Since the cut-out range corresponding to the difference is set as described above, it is possible to obtain an image in a state where a match is achieved between the reference distance RD and the subject distance WD even if the inward angle is fixed. Accordingly, the "image obtained by cutting out the cut-out range and enlarging the cut-out range at the set magnification", that is, the image that has been subjected to the electronic zoom process becomes an image with a more natural three-dimensional effect.

The medical observation apparatus 100 sets the cut-out range for each of the medical image for the right eye and the medical image for the left eye based on the reference distance RD and the subject distance WD as described with reference to FIGS. 7 to 10, for example. Then, the medical observation apparatus 100 cuts out the image in the set cut-out range and enlarges the image in the cut-out range at the set magnification.

Note that the electronic zoom process according to the present embodiment is not limited to or by the above-described examples.

For example, when the set magnification is a variable value, the medical observation apparatus 100 sets a size of the set cut-out range to a size corresponding to the set magnification. That is, the medical observation apparatus 100 can adjust the magnification according to the size of the cut-out range. For example, when the magnification is changed, the medical observation apparatus 100 changes the size of the set cut-out range to a size corresponding to the changed magnification. The medical observation apparatus 100 refers to a table (or database) in which the magnification and the size of the cut-out range are associated to identify the size of the cut-out range corresponding to the set magnification.

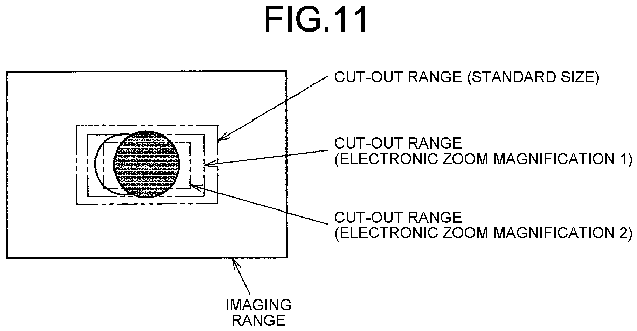

FIG. 11 is an explanatory view for describing an example of the electronic zoom process in the medical observation apparatus 100 according to the present embodiment, and illustrates an example of the size of the cut-out range corresponding to the magnification.

In addition, the medical observation apparatus 100 may reset the cut-out range each time the relationship between the reference distance RD and the subject distance WD changes. Even if the subject distance WD changes, the image that has been subjected to the electronic zoom process can be made to be an image with a more natural three-dimensional effect by resetting the cut-out range.

Furthermore, when the relationship between the reference distance RD and the subject distance WD does not change, the medical observation apparatus 100 may move the cut-out range based on a predetermined moving operation. The medical observation apparatus 100 moves the cut-out range in a state of maintaining the correspondence between cut-out ranges set for a medical image for the right eye and a medical image for the left eye.

When the cut-out range is moved in the state of maintaining the set correspondence between the set cut-out range, the cut-out range is moved in the state of maintaining the match between the reference distance RD and the subject distance WD. Accordingly, the image that has been subjected to the electronic zoom process becomes an image with a more natural three-dimensional effect even if the cut-out range is moved in the state of maintaining the correspondence between the cut-out ranges.

Here, examples of the predetermined moving operation according to the present embodiment include any operations such as an "operation by the line of sight", an "operation by a gesture", an "operation by voice", an "operation on a remote controller such as a foot switch and a hand switch", and an "operation on an any operation device provided in the medical observation apparatus 100".

When the moving operation is the operation by the line of sight, the medical observation apparatus 100 identifies the content of the operation by the line of sight based on, for example, a "detection result of the line of sight detected by any image processing on a captured image on which a detection target of the operation has been captured". An imaging device that captures the detection target of the operation may be an imaging device provided in the medical observation apparatus 100, or may be an imaging device outside the medical observation apparatus 100 such as a surgical site camera. In addition, the image processing related to the detection of the line of sight may be performed by the medical observation apparatus 100 or may be performed by an external device of the medical observation apparatus 100.

When the move operation is the operation by a gesture, the medical observation apparatus 100 identifies the content of the operation by the gesture based on, for example, a "detection result of the gesture detected by any image processing on a captured image on which a detection target of the operation has been captured". In addition, the image processing related to the detection of the gesture may be performed by the medical observation apparatus 100 or may be performed by an external device of the medical observation apparatus 100.

When the moving operation is the operation by voice, the medical observation apparatus 100 identifies the content of the operation by voice based on, for example, a "detection result of predetermined voice detected by any signal processing on the voice obtained by a voice input device such as a microphone". The voice input device may be a voice input device provided in the medical observation apparatus 100 or a voice input device outside the medical observation apparatus 100. In addition, the signal processing on the voice may be performed by the medical observation apparatus 100 or may be performed by an external device of the medical observation apparatus 100.

When the moving operation is the operation on a remote controller, or the operation on an operation device provided in the medical observation apparatus 100, the medical observation apparatus 100 identifies the content of the operation based on, for example, an operation signal corresponding to the operation.

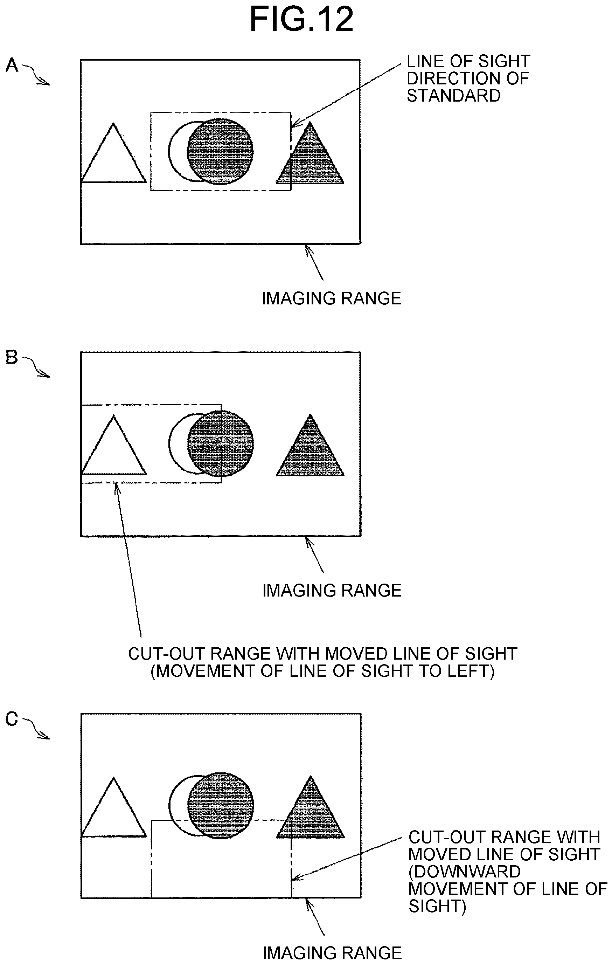

FIG. 12 is an explanatory view for describing an example of the electronic zoom process in the medical observation apparatus 100 according to the present embodiment, and illustrates an example of movement of a cut-out range based on the moving operation by the line of sight. A of FIG. 12 illustrates an example of the cut-out range set before the moving operation by the line of sight is performed. B of FIG. 12 illustrates an example of the cut-out range set when the moving operation by the line of sight has been performed, and illustrates an example of the cut-out range set when the line of sight has moved to the left. C of FIG. 12 illustrates another example of the cut-out range set when the moving operation by the line of sight has been performed, and illustrates an example of the cut-out range set when the line of sight has moved downward.

### [3-2] Display Control Process According to Present Embodiment

Next, the display control process according to the present embodiment will be described. The display control process is, for example, a process of displaying a medical image on a display screen. Note that it goes without saying that the medical observation apparatus 100 can display an image other than the medical image on the display screen.

Examples of the medical image displayed on the display screen by the medical observation apparatus 100 include the following examples.
- One or both of a medical image for the right eye and a medical image for the left eye
- Image obtained by performing the electronic zoom process on each of the medical image for the right eye and the medical image for the left eye.
- Combination of the above examples

The medical observation apparatus 100 switches the medical image to be displayed on the display screen, for example, based on a display switching operation performed by a user of the medical observation apparatus 100 or automatically based on a result of image processing.

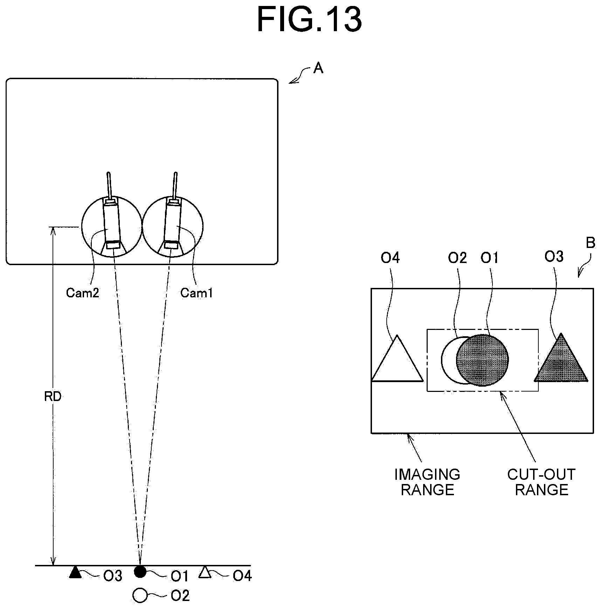

FIG. 13 is an explanatory view for describing an example of the display control process in the medical observation apparatus 100 according to the present embodiment. A of FIG. 13 illustrates a case where subjects O1, O2, O3, and O4 are captured in the state illustrated in A of FIG. 7. B of FIG. 13 illustrates a medical image for the left eye obtained in the case of A of FIG. 13.

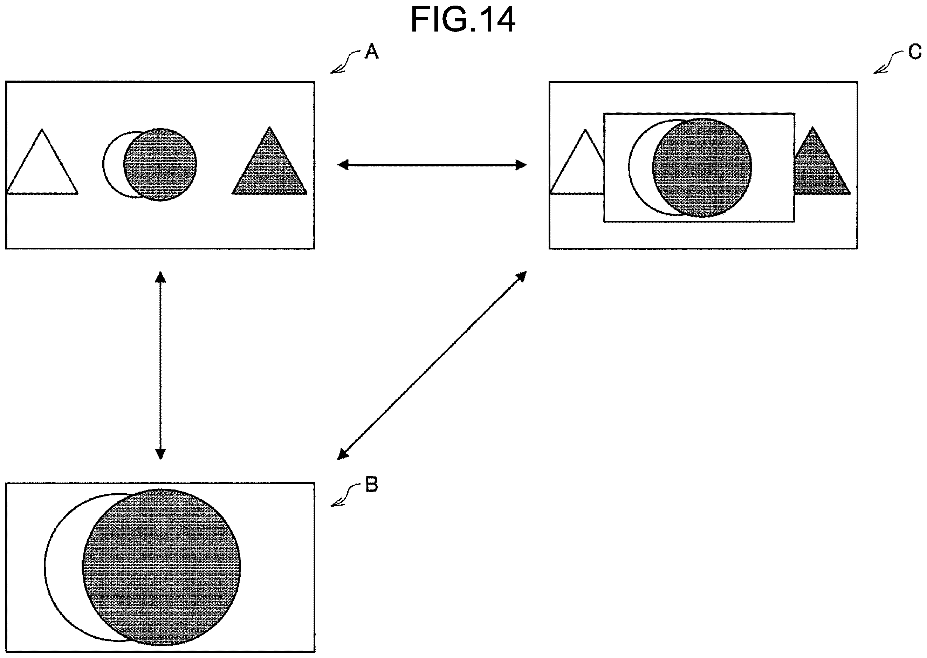

Hereinafter, an example of display performed by the display control process will be described with reference to the example illustrated in FIG. 13. FIG. 14 is an explanatory view for describing a first example of the display performed by the display control process in the medical observation apparatus 100 according to the present embodiment.

A of FIG. 14 illustrates an example in which one or both of the medical image for the right eye and the medical image for the left eye are displayed on the display screen. In the case of the example illustrated in A of FIG. 14, a display range of the medical image displayed on the display screen is an image corresponding to an angle of view of an imaging device. Examples of the angle of view of the imaging device may include an "angle of view that is the same as a size of a real scale of a subject viewed with the naked eye when an observer observes the display screen of the display device at an optimum viewing distance". That is, each of the first imaging device and the second imaging device has, for example, the "angle of view at which the size of the subject included in the medical image when the observer observes the display screen on which the medical image is displayed at a predetermined viewing distance, such as the optimum viewing distance, is similar to the size seen when an observer views the real scale of the subject with naked eyes.

B of FIG. 14 illustrates an example in which the image that has been subjected to the electronic zoom process is displayed on the display screen. The display illustrated in B of FIG. 14 is, for example, display used when an operator (an example of the observer) desires to observe an enlarged image of a region to be treated during a treatment in a highlighted manner.

Note that it is difficult for the operator (the example of the observer) to observe a periphery of a cut-out range when only the image that has been subjected to the electronic zoom process is displayed on the display screen, for example, as illustrated in B of FIG. 14. Therefore, for example, when a hand and a tool of an assistant unexpectedly approach, there is a possibility that the operator may notice approaching of these, and as a result, safety in medical practice is likely to be affected. Therefore, it is considered that the display illustrated in B of FIG. 14 is desirably "display that is temporarily performed based on the operator's intentional operation".

C of FIG. 14 illustrates an example in which "one or both of the medical image for the right eye and the medical image for the left eye" and "the image that has been subjected to the electronic zoom process" are displayed in combination on the display screen. C of FIG. 14 is an "example of display in which the image that has been subjected to the electronic zoom process is superimposed on one or both of the medical image for the right eye and the medical image for the left eye".

When the display illustrated in C of FIG. 14 is performed, not only an image of a portion that the operator (the example of the observer) desires to view in an enlarged manner but also an image around the cut-out range is displayed. Accordingly, when the display illustrated in C of FIG. 14 is performed, it is possible to improve safety in medical practice as compared with the case where the display illustrated in B of FIG. 14 is performed.

When the display illustrated in C of FIG. 14 is performed, it is considered that "display in which one of the medical image for the right eye and the medical image for the left eye is displayed, and the image that has been subjected to the electronic zoom process is displayed as a 3D image" provides an image that is more natural for the observer. Note that it goes without saying that "display in which both the medical image for the right eye and the medical image for the left eye are displayed as 3D images, and the image that has been subjected to the electronic zoom process is also displayed as a 3D image" may be performed when the display illustrated in C of FIG. 14 is performed.

In addition, when the display illustrated in C of FIG. 14 is performed, the medical observation apparatus 100 may perform adjustment such that a display parallax of the image that has been subjected to the electronic zoom process is seen on the farther side based on a distance from the observer to an observation target. The distance from the observer to the observation target can be obtained by, for example, measuring the distance with a distance sensor or adding the optimum viewing distance of the display screen of the display device to the subject distance WD. Since the display parallax is adjusted as described above, the image that has been subjected to the electronic zoom process becomes an image that gives a feeling as if the observation target is viewed from the position of the observer.

Note that a method of setting the image that has been subjected to the electronic zoom process to be the image that gives the feeling as if the observation target is viewed from the position of the observer is not limited to the above example. For example, the medical observation apparatus 100 can also "set the image that has been subjected to the electronic zoom process to be the image that gives the feeling as if the observation target is viewed from the position of the observer" by setting the magnification related to the electronic zoom process.

For example, when a size of the display screen of the display device and the optimum viewing distance on the display screen of the display device do not change, a subject included in the image that has been subjected to the electronic zoom process can be recognized to be located on the farther side by the observer by increasing a display parallax amount within an allowable range. Here, examples of the allowable range of the display parallax amount may include a range that does not exceed a representative value of the human's interocular distance. In addition, when an image of a set cut-out range is enlarged by an electronic zoom, the display parallax amount in the enlarged image of the cut-out range increases as the magnification related to the electronic zoom process increases.

Accordingly, the medical observation apparatus 100 sets the magnification related to the electronic zoom process such that the display parallax amount in the enlarged image of the cut-out range (hereinafter, referred to as a "first display parallax amount") approaches a display parallax amount corresponding to the distance from the observer viewing the display screen to the subject (hereinafter referred to as a "second display parallax amount"). Here, the state where "the first display parallax amount approaches the second display parallax amount" includes, for example, a state where "the first display parallax amount matches with the second display parallax amount" and a state where "the first display parallax amount falls within the allowable range of the display parallax amount, and an absolute value of a difference between the second display parallax amount and the first display parallax amount is the minimum value".

Since the magnification related to the electronic zoom process is set such that the first display parallax amount approaches the second display parallax amount, it is possible to implement "setting the image that has been subjected to the electronic zoom process to be the image that gives the feeling as if the observation target is viewed from the position of the observer".

To give a specific example of the processing, the medical observation apparatus 100 calculates the second display parallax amount based on, for example, the angle of view of each of the first imaging device and the second imaging device, the distance from the observer to the observation target, and the size of the display screen of the display device. The medical observation apparatus 100 determines the first display parallax amount based on the calculated second display parallax amount and an upper limit value corresponding to the allowable range of the display parallax amount. Then, the medical observation apparatus 100 sets the magnification related to the electronic zoom process based on a size of the set cut-out range and the determined first display parallax amount. The medical observation apparatus 100 identifies the magnification related to the electronic zoom process by, for example, "referring to a table (or database) in which the size of the cut-out range, the display parallax amount, and the magnification are associated with each other", performing "an operation of an arbitrary algorithm that can identify the magnification based on the size of the cut-out range and the display parallax amount", or the like, and sets the identified magnification. Note that it goes without saying that the example of the "process of setting the magnification related to the electronic zoom process such that the first display parallax amount approaches the second display parallax amount" is not limited to the above example.

Note that examples of the display performed by the display control process according to the present embodiment are not limited to the examples described with reference to FIGS. 13 and 14.

For example, the medical observation apparatus 100 can display an image of a cut-out range corresponding to a set magnification.

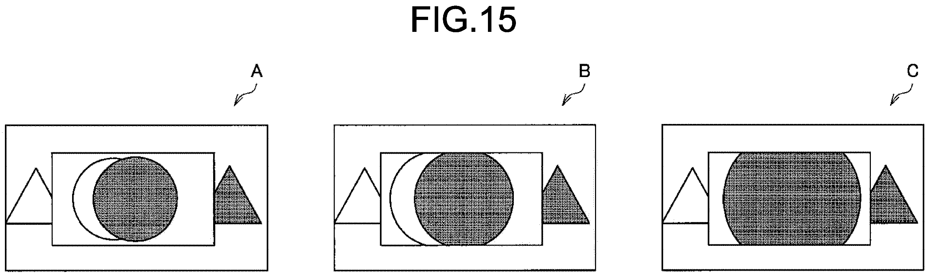

FIG. 15 is an explanatory view for describing a second example of display performed by the display control process in the medical observation apparatus 100 according to the present embodiment, and illustrates an example of the display when a size of a cut-out range corresponding to a magnification is set in a zoom process.

A of FIG. 15 illustrates an example of display when the cut-out range set in the zoom process is a "cut-out range (standard size)" illustrated in FIG. 11. B of FIG. 15 illustrates an example of display when the cut-out range set in the zoom process is a "cut-out range (electronic zoom magnification 1)" illustrated in FIG. 11. C of FIG. 15 illustrates an example of display when the cut-out range set in the zoom process is a "cut-out range (electronic zoom magnification 2)" illustrated in FIG. 11.

In addition, when the cut-out range moves according to a predetermined moving operation in the zoom control, it is possible to display the image of the cut-out range so as to correspond to the movement of the cut-out range, for example, as in the example illustrated below. Note that it goes without saying that the example of the display corresponding to the movement of the cut-out range is not limited to the example illustrated below.

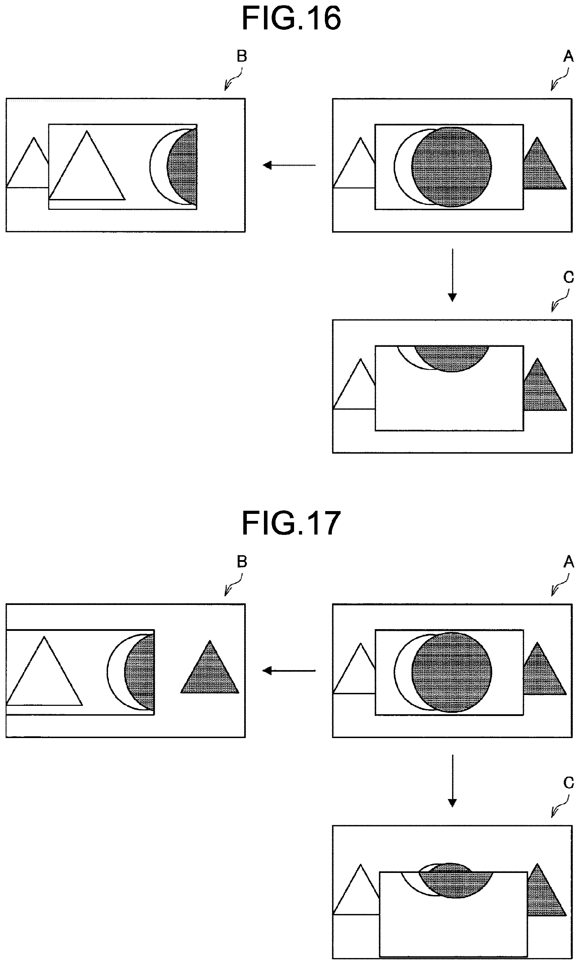

FIG. 16 is an explanatory view for describing a third example of display performed by the display control process in the medical observation apparatus 100 according to the present embodiment, and illustrates an example of the display when the cut-out range is moved in response to a predetermined moving operation in the zoom process.

A of FIG. 16 illustrates an example of display when the cut-out range is the cut-out range illustrated in A of FIG. 12. B of FIG. 16 illustrates an example of display when the cut-out range is the cut-out range illustrated in B of FIG. 12. C of FIG. 16 illustrates an example of display when the cut-out range is the cut-out range illustrated in C of FIG. 12.

In the display example illustrated in FIG. 16, an image within the moved cut-out range is displayed in a display area in a state where the display area in which the electronically zoomed image is displayed is fixed on the display screen. For example, when the display illustrated in FIG. 16 is performed, an observer can perform observation by moving only the image enlarged by the electronic zoom (electronically zoomed image) while maintaining the observation state around the cut-out range.

FIG. 17 is an explanatory view for describing the third example of display performed by the display control process in the medical observation apparatus 100 according to the present embodiment, and illustrates another example of the display when the cut-out range is moved in response to a predetermined moving operation in the zoom process.

A of FIG. 17 illustrates another example of display when the cut-out range is the cut-out range illustrated in A of FIG. 12. B of FIG. 17 illustrates another example of display when the cut-out range is the cut-out range illustrated in B of FIG. 12. C of FIG. 17 illustrates another example of display when the cut-out range is the cut-out range illustrated in C of FIG. 12.

In the display example illustrated in FIG. 17, a position of a display area where the electronically zoomed image is displayed on the display screen is moved in a moving direction of the cut-out range so as to be interlocked with the movement of the cut-out range. In the display example illustrated in FIG. 17, an image around the cut-out range is hidden as the display area moves, but an image around the cut-out range other than the moving direction remains visible. In addition, since the display area moves in the movement direction of the cut-out range in the display example illustrated in FIG. 17, there is an "advantage that the observer feels as if he or she is moving the electronically zoomed image in a natural sense".

### [4] Example of Effect Achieved by Using Medical Observation Apparatus According to Present Embodiment

By using the medical observation apparatus according to the present embodiment, for example, the following effects can be achieved. Needless to say, the effects achieved by using the medical observation apparatus according to the present embodiment are not limited to the following examples.
- The medical observation apparatus according to the present embodiment performs the electronic zoom process on the medical images captured by at least two imaging devices with the fixed inward angle, thereby obtaining the image achieving the match between the reference distance and the subject distance. Accordingly, the medical observation apparatus according to the present embodiment can obtain the medical image with the more natural three-dimensional effect while suppressing the increase in size, weight, and cost. In addition, the observer who sees the medical image displayed on the display screen of the display device can perform observation with the natural three-dimensional effect.
- Since devices used at medical sites require sterilization, not only a closed structure is adopted, but also a structure that can be washed at the time of contamination with bacteria is required. In the medical observation apparatus according to the present embodiment, at least two imaging devices with the fixed inward angle are covered with the detachable cover member, and thus, the sterilization can be supported.
- Since it is possible to change the magnification and move the cut-out range according to the predetermined moving operation in the electronic zoom process, there is no problem of the increase in size, weight, and cost of the device caused by the change of magnification and movement of cut-out range.
- Since the angle of view of the imaging device is set to be the "angle of view that is the same as the size of the real scale of the subject viewed with the naked eye when the observer observes the display screen of the display device at the optimum viewing distance", the operator such as a surgeon can proceed with the operation in a state where motion of the surrounding hand or the like is visible. In addition, the surgeon can observe the affected site in 3D in an enlarged manner in detail in order to perform a delicate procedure, and at the same time, proceed with the surgery while ensuring the safety.

### (Program According to Present Embodiment)

When a program (for example, a program capable of executing processing in the medical observation apparatus according to the present embodiment, such as a "program that functions as the image processor 110 illustrated in FIG. 6" and a "program that functions as the image processor 110 and the display controller 112 illustrated in FIG. 6") for causing a computer system to function as the medical observation apparatus according to the present embodiment is executed by a processor or the like in the computer system, it is possible to obtain a medical image with a more natural three-dimensional effect. The computer system according to the present embodiment includes a single computer or a plurality of computers. A series of processes according to the control method of the present embodiment is performed by the computer system according to the present embodiment.

In addition, programs for causing the computer system to function as the medical observation apparatus according to the present embodiment are executed by a processor or the like in the computer system, thereby enabling the foregoing advantageous effects afforded by the processing pertaining to the medical observation apparatus according to the present embodiment to be provided.

As described above, the preferred embodiments of the present disclosure have been described in detail with reference to the accompanying drawings, but the technical scope of the present disclosure is not limited to such examples. It is apparent that a person skilled in the art of the technical field of the present disclosure can make various changes or modifications within the scope of the technical concept described in the claims, and it is naturally understood that these also belong to the technical scope of the present disclosure.

For example, in the above description, the program (computer program) for causing the computer system to function as the medical observation apparatus according to the present embodiment is provided, but the present embodiment can further provide a recording medium storing the program therein.

The configuration described above illustrates an example of the present embodiment, and naturally belongs to the technical scope of the present disclosure.

In addition, the effects described in the present description are only illustrative or exemplary and not restrictive. That is, the technology according to the present disclosure can achieve other effects that are apparent to those skilled in the art from the description of the present description, in addition to or in place of the above effects.

Note that the following configuration also belongs to the technical scope of the present disclosure.

### Reference Signs List

- 100: MEDICAL OBSERVATION APPARATUS
- 102: IMAGING UNIT
- 104: DISPLAY UNIT
- 106: CONTROL UNIT
- 108: IMAGING CONTROLLER
- 110: IMAGE PROCESSOR
- 112: DISPLAY CONTROLLER

## Claims

1. A medical image processing apparatus comprising:
an image processor configured to set a cut-out range for each of a medical image for a right eye and a medical image for a left eye, captured respectively by a first imaging device and a second imaging device with a fixed inward angle that is an angle formed by imaging directions, and perform an electronic zoom process of enlarging an image in the cut-out range; and
a display controller configured to control display on a display screen of the medical image for the right eye and the medical image for the left eye that have been subjected to the electronic zoom process by the image processor, wherein
the image processor is configured to set the cut-out range for each of the medical image for the right eye and the medical image for the left eye based on a reference distance corresponding to the inward angle and a subject distance in the first imaging device and the second imaging device.

2. The medical image processing apparatus according to claim 1, wherein the image processor is configured to enlarge the image in the cut-out range at a set magnification.

3. The medical image processing apparatus according to claim 2, wherein the image processor sets a size of the cut-out range to a size corresponding to the magnification.

4. The medical image processing apparatus according to claim 2, wherein the image processor is configured to set the magnification such that a display parallax amount in the enlarged image of the cut-out range approaches a display parallax amount corresponding to a distance from an observer viewing the display screen to a subject.

5. The medical image processing apparatus according to claim 1, wherein the image processor is configured to acquire a distance measured by a distance sensor or calculates a hyperfocal distance in the first imaging device and the second imaging device to identify the subject distance.

6. The medical image processing apparatus according to claim 1, wherein the image processor is configured to set the cut-out range based on a difference between the reference distance and the subject distance.

7. The medical image processing apparatus according to claim 6, wherein when the difference is zero, the image processor is configured to set the cut-out range such that a center position of the cut-out range becomes a center position of each of the medical image for the right eye and the medical image for the left eye.

8. The medical image processing apparatus according to claim 6, wherein when the difference is other than zero, the image processor is configured to set the cut-out range such that a center position of the cut-out range is moved in a horizontal direction by pixels corresponding to the difference from a center position of each of the medical image for the right eye and the medical image for the left eye.

9. The medical image processing apparatus according to claim 1, wherein the image processor is configured to reset the cut-out range each time a relationship between the reference distance and the subject distance changes.

10. The medical image processing apparatus according to claim 1, wherein
when a relationship between the reference distance and the subject distance does not change,
the image processor is configured to move the cut-out range in a state of maintaining a correspondence between the cut-out ranges respectively set for the medical image for the right eye and the medical image for the left eye based on a predetermined moving operation.

11. The medical image processing apparatus according to claim 1, further comprising a display controller configured to perform a display control process of displaying the medical image on the display screen.

12. The medical image processing apparatus according to claim 11, wherein the display controller is configured to display an image, obtained by performing the electronic zoom process on each of the medical image for the right eye and the medical image for the left eye, on the display screen.

13. The medical image processing apparatus according to claim 11, wherein the display controller is configured to display, on the display screen, one or both of the medical image for the right eye and the medical image for the left eye and an image obtained by performing the electronic zoom process on each of the medical image for the right eye and the medical image for the left eye.

14. The medical image processing apparatus according to claim 13, wherein the display controller is configured to superimpose the image that has been subjected to the electronic zoom process on the one or both of the medical image for the right eye and the medical image for the left eye, and display the superimposed image.

15. The medical image processing apparatus according to claim 11, wherein the display controller is configured to display one or both of the medical image for the right eye and the medical image for the left eye on the display screen.

16. The medical image processing apparatus according to claim 1, wherein each of the first imaging device and the second imaging device has an angle of view at which a size of a subject included in a medical image when an observer observes the display screen on which the medical image is displayed at a predetermined viewing distance is similar to a size of a real scale of the subject viewed by the observer with naked eyes.

17. The medical image processing apparatus according to claim 1, further comprising an imaging unit including the first imaging device capturing the medical image for the right eye and the second imaging device capturing the medical image for the left eye, and has a fixed inward angle that is an angle formed by an imaging direction in the first imaging device and an imaging direction in the second imaging device.

18. The medical image processing apparatus according to claim 1, further comprising a display unit including a display device configured to display the medical images on the display screen.
